# EUROPEAN PATENT SPECIFICATION

(11) **EP 2 927 334 B1**
(45) Date of publication and mention of the grant of the patent: **06.03.2019**
(21) Application number: 14162873.5
(22) Date of filing: 31.03.2014
(51) Int. Cl.: C22C 14/00, C22C 1/10, C22F 1/18, A61L 27/06, A61L 27/12, A61L 27/50, A61L 27/58, A61L 31/02, A61L 31/14

(54) **Titanium based ceramic reinforcement alloy for use in medical implants**
Titanbasierte Keramikverstärkungslegierung zur Verwendung in medizinischen Implantaten
Alliage de renforcement céramique à base de titane destiné à être utilisé dans des implants médicaux

(43) Date of publication of application: 07.10.2015
(73) Proprietor: Pulse IP, LLC, Quakertown, PA 18951 (US)
(72) Inventor: Kulak, Leonid, 03142 Kiev (UA); Firstov, Sergei, Kiev (UA); Kuzmenko, Mykola, Kiev (UA); Demchyshyn, Anatolii, Kiev (UA); Fisk, Andrew E., Philadelphia, PA Pennsylvania 19118 (US)
(74) Representative: SSM Sandmair

(56) References cited:
- EP-A1- 0 555 033
- EP-A2- 2 021 041
- KR-A- 20080 072 812
- US-B2- 6 752 882
- TAVARES A M G ET AL: "The addition of Si to the Ti-35Nb alloy and its effect on the corrosion resistance, when applied to biomedical mater", JOURNAL OF ALLOYS AND COMPOUNDS, vol. 591, 3 January 2014 (2014-01-03), pages 91-99, XP028605334, ISSN: 0925-8388, DOI: 10.1016/J.JALLCOM.2013.12.183
- KEE-DO WOO ET AL: "Microstructure and Biocompatibility of Ti-Nb-Si-HA Composites Fabricated by Rapid Sintering Using HEMM Powders", KOREAN JOURNAL OF MATERIALS RESEARCH, vol. 23, no. 7, 27 July 2013 (2013-07-27), pages 353-358, XP055118954, ISSN: 1225-0562, DOI: 10.3740/MRSK.2013.23.7.353
- VOZILKIN V A ET AL: "Precipitation of silicides and germanides in titanium alloys", FIZIKA METALLOV I METALLOVEDENIE, AKADEMIJA NAUK SSSR, RU, no. 4, 1 January 1990 (1990-01-01), pages 152-158, XP009178080, ISSN: 0015-3230
- R. Betner D. ET AL: "Titanium and Titanium Alloys" In: "Metals Handbook (Ninth edition) Vol. 3", 1 January 1980 (1980-01-01), American Society for Metals, OHIO, US, XP055448285, ISBN: 978-0-87170-009-4 page 357,

## Description

The invention relates to a titanium based, ceramic reinforced alloy ingot for use in producing medical implants. More particularly, the invention pertains to a ceramic reinforced alloy ingot comprising titanium, niobium and silicon. The alloy has both an α crystal phase and a β crystal phase. The ingot has an ultimate tensile strength of about 940 MPa or more, and a Young's modulus of about 150 GPa or less.

There is a great commercial interest in the production of biocompatible, medically suitable implants for surgically jointing bone and implanting teeth. Medical implants such as screws, pins, rods, bars, springs, coils, cables, staples, clips, plates and the like require materials with very high tensile strength and high cyclic fatigue life while also having a modulus of elasticity low enough to be compatible with bone. Common alloys include titanium, stainless steel and cobalt chrome alloys. Stainless steel and cobalt chrome alloys exhibit very high tensile strength, but both contain nickel and chromium which are known irritants to the body. In addition, these alloys have low ductility and a Young's modulus approaching five times that of bone. This high tensile strength and Young's modulus also makes it difficult to machine these components cost effectively using conventional techniques. Titanium and its alloys are especially popular choices for orthopedic bone screws and plates commonly used for spinal fixation.

Titanium alloys for a variety of applications are known in the art and there are numerous literature references disclosing a wide range of elements which are used to provide alloys having desired characteristics, such as increased tensile strength and ductility. Generally, titanium and its alloys may exist in one or a mixture of two basic crystalline structures, namely the α phase, which is a hexagonal close-packed structure, and the β phase which is a body-centered cubic structure. The commercially pure grades of titanium alloys have low tensile strengths but show no signs of tissue irritation. These alloys are commonly used for orthopedic plates which are implanted externally to the bone structure and can therefore have a larger size. Ti₆AlV₄ alloys are commonly used for higher strength applications such as fixation screws or plates which must be contained in a small area. One known medically implantable alloy is disclosed in U.S. patent 6,752,882. It provides a biocompatible low modulus, high strength titanium-niobium alloy containing α phase as a major phase and consisting essentially of 10-30 wt.% of Nb and the balance titanium. U.S. patent 5,954,724 relates to titanium alloys suitable for use for medical implants and devices having a high-strength, low-modulus, and high hardness with improved corrosion resistance due to the addition of hafnium and molybdenum, and which additionally allow for surface hardening of an implant made of this alloy. U.S. patent 7,892,369 provides a method for modifying the microstructure of titanium alloys for use in the manufacture of orthopedic prostheses. An orthopedic prosthesis is initially formed from a titanium alloy and subsequently subjected to a thermal treatment followed by rapid quenching. The microstructure of the titanium alloy in the prosthesis has improved resistance to fretting fatigue. U.S. patent 7,682,473 provides an implant prosthesis composed of a TiAINb alloy having a modulus near that for bone to prevent stress shielding, and a tensile and compressive strength and fracture toughness equal to or greater than that of bone. A key problem with other alloys which use aluminum and vanadium is the suspected effect of Al and V when movement and fretting are involved. The release of Al and V into the blood stream could cause irritation for the patient in the long term. Another issue with certain grades of titanium is the so called "notch effect" during cyclic fatigue. Prepared and polished samples of certain titanium alloys have been shown to have fatigue strength near the ultimate tensile strength. However, when a notch is introduced to the sample, the fatigue strength can be lowered to 40% of the ultimate tensile strength. Since implantable devices must be laser marked with the appropriate tracking information, a notch situation always exists and care must be taken not to exceed the notch fatigue strength.

The problems associated with designing an implantable device are specifically, providing an alloy with high tensile strength, and a marginal Young's modulus that contains no known irritants which can be economically machined with conventional methods. The present invention addresses all these issues. The invention provides an alloy of titanium, niobium and silicon. Titanium and niobium alloys are known to form alloys with very low Young's modulus (50-80 GPa).

In A.M.G. Tavares et.al.: "The addition of Si to the Ti-35Nb alloy and its effect on the corrosion resistance, when applied to biomedical matter", Journal of Alloys and Compounds, vol. 591, pages 91-99, XP028605334, ISSN: 0925-8388, DOI: 10.1016/J.JALLCOM.2013.12.183, the corrosion resistance of alloys comprising 25 mass% Nb, various amounts of silicon and the balance titanium when in physiological medium is assessed. The amount of silicon varies from 0 to 0.15 to 0.35 and to 0.55 mass%. Mechanical and corrosion property, wear resistance and biocompatibility of Ti-Nb-Si-hydroxyapatite (HA) composites are reported in Kee-Do Woo et.al.: Microstructure and Biocompatibility of Ti-Nb-Si-HA Composites Fabricated by Rapid Sintering Using HEMM Powders", Korean Journal of Materials Research, vol. 23, no. 7, 27 July 2013 (2013-007-27), pages 353-358, XP055118954, ISSN: 1225-0562, DOI: 10.3740/MRSK.2013.23.7.353. Those properties were specifically observed for Ti-26wt.%Nb-1%Si-10%HA. VA. Vozilkin et.al.: "Precipitation of silicides and germanides in titanium alloys", Fizika Metallov i Metallovedenie, Akademija Nauk SSSR, RU, no. 4, 1 January 1990 (1990-01-01), pages 152-158, XP009178080, ISSN: 0015-3230 discusses microstructural changes in titanium based alloys, in particular, in Ti-(10-35)%Nb-(0.5-1)%Si.

EP 2 021 041 A2 discloses a medical bone implant which consists at least partly of a biocompatible titanium alloy with high strength and a low module of elasticity. The titanium alloy comprises niobium in a proportion of between 25.0 mass% and 30.0 mass%, iron and/or manganese in a proportion of between 0.5 mass% and 3.0 mass%, silicon in a proportion of between 0.1 mass% and 1.0 mass%, and the remainder of at least 66.0 mass% titanium including unavoidable impurities. High strength and a low module of elasticity are reported once the alloy has been brought by cold forming into a martensitic structural state.

KR 20080072812 A is concerned with titanium based alloys for treating the root canal of a tooth. The alloys comprise titanium, niobium and either silicon or germanium. Based on considerations concerning the bond order and the metal d-orbital energy level two Ti-Nb-Si-alloys are proposed, namely, Ti-26 wt.% Nb-0.5 wt.% Si and Ti-26 wt.% Nb-1wt. %Si.

It is an object of the invention to provide an ingot comprising a titanium based alloy with sufficient strength for the manufacture of orthopedic devices such as bone plates and fixation screws.

This invention overcomes the limitations of conventional alloys by including within a solid solution of the metals, a glassy silicon ceramic which acts to absorb energy during crack propagation and retard dislocations during applied stress. The atomic percent of this glassy silicon ceramic is controlled as to still allow for a moderately low Young's modulus and good formability. The inventive alloy of primarily Ti with the addition of Nb and Si produces alloys which have a complex alpha/beta structure with an amount of glassy material. The resulting alloy has a higher strength then the titanium grades presently used in medical implants while retaining a comparable elastic modulus.

The invention provides an ingot according to claim 1 and a method according to claim 10. The preferred embodiments are in claims 2-9, 11-13.

An alloy is formed by combining commercially pure quantities of titanium, niobium and silicon. These may be obtained in the form of bars, wires, powders, particles, or any other convenient form. These are then heated until each is molten and blended into a substantially uniform admixture. The amount of titanium may range from about 61.5 wt.% to about 94.5 wt.%, preferably from about 72.5 wt.% to about 92 wt.%, and more preferably from about 78 wt.% to about 88.75 wt.%. The amount of niobium may range from about 5 wt. % to about 35 wt. %, preferably from about 7 wt. % to about 25 wt. %, and more preferably from about 10 wt.% to about 20 wt.%. The amount of silicon may range from about 0.5 wt. % to about 3.5 wt. %, preferably from about 1 wt. % to about 2.5 wt. %, and more preferably from about 1.25 wt.% to about 2 wt.%. The alloy has no more than 2 wt.% of nitrogen, oxygen, or carbon. More preferably the alloy has about 1 wt.% or less of nitrogen, oxygen or carbon. Still more preferably the alloy has about 0.5 wt.% or less of nitrogen, oxygen or carbon.

A method for preparing such a high strength, low modulus, biocompatible titanium alloy involves mechanically blending the above components, and then heating them until melted, one or more times.

The alloys are preferably made by mechanically blending accurately weighed portions of the pure elements and melting the blend in a furnace such as a plasma arc furnace or vacuum arc furnace, and re-melting as necessary to achieve uniformity, and then casting and cooling. One example of a method of melting includes combining the components in a commercially available arc-melting vacuum pressure casting system. A melting chamber is first evacuated and purged with an inert gas such as argon. An argon pressure of, for example 1.5 kgf/cm² may be maintained during melting. The appropriate amounts of titanium, niobium and silicon are prepared by electron beam skull melting with induction stirring of the melt. The resulting mixture may optionally be re-melted multiple times to improve homogeneity. The molten alloy is then cast, or drawn out of the crucible by a water-cooled rod to form a cylindrical ingot, with cooling. The alloy has a combination crystal lattice structure of both α and β phases. In particular, the alloy has a hexagonal crystal lattice α phase of from about 20 vol.% to about 70 vol. %, and a cubic body centered β crystal lattice phase of from about 30 vol.% to about 80 vol.%. Preferably the alloy has a hexagonal crystal lattice a phase of from about 40 vol.% to about 70 vol.%, and a cubic body centered β crystal lattice phase of from about 30 vol.% to about 60 vol.%. More preferably the alloy comprises a hexagonal crystal lattice α phase of from about 45 vol.% to about 65 vol.%, and a cubic body centered β crystal lattice phase of from about 45 vol. % to about 60 vol.%.

The resulting ingot has an ultimate tensile strength of about 940 MPa or more, usually from about 1000 MPa to about 1400 MPa, and more usually from about 1100 MPa to about 1300 MPa. The resulting ingot has a Young's modulus of about 150 GPa or less, usually from about 100 GPa to about 150 GPa, and more usually from about 110 GPa to about 140 GPa.

The resulting ingot may then be formed into the desired medial implant shape, such as those in the form of a screw, pin, rod, bar, spring, coil, cable, staple, clip, plate, or the like. The implant may also be form into customized shapes such as those conforming to hip joint stems, femoral heads, knee femoral components, knee tibial components, intramedullary nails, inner ear vent tubes, spinal plates, spinal disks, pelvic plates, dental implants, cardiovascular implants, compression hip screws, and the like. Such forming may be done by the use of customary machine tooling. Optionally either the cast ingot or the machined medical implant may be annealed for additional strength, polished or anodized by well known methods. Annealing may be done by heating at temperatures ranging from about 500 °C to a bout 1200 °C, preferably from about 750 °C to about 1000 °C, for from about 20 minutes to about 360 minutes, preferably from about 40 minutes to about 120 minutes. Polishing may be done by mechanical burnishing. Anodizing may be done by electrochemically oxidizing the surface.

The following non-limiting examples serve to illustrate the invention.

### EXAMPLES

Three alloys were formed and tested in both the as cast condition, and after annealing at 950 °C for 1 hour in vacuum. The alloys were prepared by electron beam skull melting with induction stirring of the melt. The resulting material was drawn out of the crucible by a water cooled rod to form a cylindrical ingot.

| Alloy Test | Condition | Nb | Si | UTS (Mpa) | Young's Modulus (GPa) | Yield Strength (Mpa) | Elongation (%) |
|---|---|---|---|---|---|---|---|
| 1 | As Cast | 10 | 1.1 | 1012 | 113 | 940 | 4.6 |
| 2 | As Cast | 13 | 1.5 | 995 | 112 | 937 | 6.2 4.8 |
| 3 | As Cast | 21 | 1.25 | 1022 | 110 | 960 | |
| | | | | | | | |
| 1a | Annealed | 10 | 1.1 | 1006 | 81 | 945 | 4.5 |
| 2a | Annealed | 13 | 1.5 | 1008 | 83 | 957 | 3.3 |
| 3a | Annealed | 21 | 1.25 | 957 | 76 | 850 | 3.25 |

The sample ingots were subjected to machinability tests, polishing tests and color anodizing. The composition performed excellently in all cases, with the polishing and anodizing exceeding the characteristics of commercially available Grade 4 and Grade 23 titanium. Detailed chemical and phase analysis of the Ti-21 Nb-1.25Si material was performed. The phase analysis shows a roughly 55/45 alpha/beta structure. XPS analysis confirmed that there existed a large number of atoms in a glassy phase with 1.6 at% of the material existing as SiC. This SiC glassy ceramic is deposited at the grain boundaries. Given the high fracture toughness of the carbide these interstitial components act not only to prohibit dislocation movement but also absorb energy in the case of crack propagation. The presence of carbon in the alloy used to form the SiC is present in the starting raw material as a typical impurity.

## Claims

1. An ingot comprising an alloy, the alloy comprising from 5 wt.% to 35 wt.% of niobium, from 0.5 wt.% to 3.5 wt.% of silicon and from 61.5 wt.% to 94.5 wt.% of titanium as the balance, the alloy having no more than 2 wt.% of nitrogen, no more than 2 wt.% of oxygen, and no more than 2 wt.% of carbon, the alloy having a hexagonal crystal lattice α phase of from 20 vol. % to 70 vol. %, and a cubic body centered β crystal lattice phase of from 30 vol.% to 80 vol.%, the ingot having an ultimate tensile strength of 940 MPa or more, and a Young's modulus of 150 GPa or less, and within the solid solution of the metals including a glassy silicon ceramic.

2. The ingot according to claim 1 wherein the alloy comprises from 10 wt.% to 20 wt.% of niobium, from 1.25 wt.% to 2 wt.% of silicon and from 78 wt.% to 88.75 wt.% of titanium.

3. The ingot according to any one of the claims 1 and 2 which has an ultimate tensile strength of from 1000 MPa to 1400 MPa, and a Young's modulus of from 100 GPa to 150 GPa.

4. The ingot according to the previous claim which has an ultimate tensile strength of from 1100 MPa to 1300 MPa, and a Young's modulus of from 110 GPa to 140 GPa.

5. The ingot according to any one of the claims 1-4 wherein the alloy has 1 wt.% of nitrogen or less, 1 wt.% of oxygen or less, and 1 wt.% of carbon or less.

6. The ingot according to claim 5 wherein the alloy has 0.5 wt.% of nitrogen or less, 0.5 wt.% of oxygen or less, and 0.5 wt.% of carbon or less.

7. The ingot according to any one of the claims 1-6 wherein the alloy comprises a hexagonal crystal lattice a phase of from 40 vol.% to 70 vol.%, and a cubic body centered β crystal lattice phase of from 30 vol.% to 60 vol.%,

8. A medical implant formed from an ingot according to any one of claims 1-7.

9. The medical implant according to claim 8 which is in the form of a screw, pin, rod, bar, spring, coil, cable, staple, clip or plate.

10. A method of forming an ingot which comprises forming a molten alloy comprising a uniform admixture of from 5 wt.% to 35 wt.% of niobium, from 0.5 wt.% to 3.5 wt.% of silicon and from 61.5 wt.% to 94.5 wt.% of titanium as the balance, the alloy having no more than 2 wt.% of nitrogen, no more than 2 wt.% of oxygen, and no more than 2 wt.% of carbon, casting the molten alloy into a shape, and then cooling the shape into an ingot, the alloy having a hexagonal crystal lattice α phase of from 20 vol.% to 70 vol.%, and a cubic body centered β crystal lattice phase of from 30 vol.% to 80 vol.%, the ingot having an ultimate tensile strength of 940 MPa or more, and a Young's modulus of 150 GPa or less, and within the solid solution of the metals including a glassy silicon ceramic.

11. The method according to claim 10 further comprising the subsequent step of forming the ingot into a medical implant in a form chosen from the group of a screw, pin, rod, bar, spring, coil, cable, staple, clip and plate.

12. The method according to claim 11 further comprising the subsequent step of annealing the medical implant.

13. The method according to any one of the claims 10-12 further comprising the subsequent step of annealing the ingot.

## Patentansprüche

1. Ingot, der eine Legierung umfasst, wobei die Legierung 5 Gew.-% bis 35 Gew.-% Niob, 0,5 Gew.-% bis 3,5 Gew.-% Silizium und als Rest 61,5 Gew.-% bis 94,5 Gew.-% Titan umfasst, wobei die Legierung nicht mehr als 2 Gew.-% Stickstoff, nicht mehr als 2 Gew.-% Sauerstoff und nicht mehr als 2 Gew.-% Kohlenstoff umfasst, wobei die Legierung eine hexagonale Kristallgitter-α-Phase von 20 Vol.-% bis 70 Vol.-% und eine kubische körperzentrierte β-Kristallgitterphase von 30 Vol.-% bis 80 Vol.-% aufweist, wobei der Ingot eine Bruch-Zugfestigkeit von 940 MPa oder mehr und einen E-Modul von 150 GPa oder weniger aufweist und in der festen Lösung der Metalle eine glasartige Siliziumkeramik enthält.

2. Ingot nach Anspruch 1, wobei die Legierung 10 Gew.-% bis 20 Gew.-% Niob, 1,25 Gew.-% bis 2 Gew.-% Silizium und 78 Gew.-% bis 88,75 Gew.-% Titan umfasst.

3. Ingot nach einem der Ansprüche 1 und 2, der eine Bruch-Zugfestigkeit von 1000 MPA bis 1400 MPa und einen E-Modul von 100 GPa bis 150 GPa aufweist.

4. Ingot nach dem vorhergehenden Anspruch, der eine Bruch-Zugfestigkeit von 1100 MPa bis 1300 MPa und einen E-Modul von 110 GPa bis 140 GPa aufweist.

5. Ingot nach einem der Ansprüche 1 bis 4, wobei die Legierung 1 Gew.-% oder weniger Stickstoff, 1 Gew.-% oder weniger Sauerstoff und 1 Gew.-% oder weniger Kohlenstoff umfasst.

6. Ingot nach Anspruch 5, wobei die Legierung 0,5 Gew.-% oder weniger Stickstoff, 0,5 Gew.-% oder weniger Sauerstoff und 0,5 Gew.-% oder weniger Kohlenstoff umfasst.

7. Ingot nach einem der Ansprüche 1 bis 6, wobei die Legierung eine hexagonale Kristallgitter-α-Phase von 40 Vol.-% bis 70 Vol.-% und eine kubische körperzentrierte β-Kristallgitterphase von 30 Vol.-% bis 60 Vol.-% aufweist.

8. Medizinisches Implantat, das aus einem Ingot nach einem der Ansprüche 1 bis 7 geformt wird.

9. Medizinisches Implantat nach Anspruch 8, welches die Form einer Schraube, eines Stifts, eines Stabs, einer Stange, einer Feder, einer Spule, eines Seils, einer Klammer, einer Schelle oder einer Platte annimmt.

10. Verfahren zur Formung eines Ingots, das folgende Schritte umfasst:
- eine geschmolzene Legierung wird geformt, die eine einheitliche Beimischung von 5 Gew.-% bis 35 Gew.-% Niob, 0,5 Gew.-% bis 3,5 Gew.-% Silizium und als Rest 61,5 Gew.-% bis 94,5 Gew.-% Titan umfasst, wobei die Legierung nicht mehr als 2 Gew.-% Stickstoff, nicht mehr als 2 Gew.-% Sauerstoff und nicht mehr als 2 Gew.-% Kohlenstoff umfasst,
- die geschmolzene Legierung wird zu einer Form gegossen, und die Form wird anschließend zu einem Ingot abgekühlt, wobei die Legierung eine hexagonale Kristallgitter-α-Phase von 20 Vol.-% bis 70 Vol.-% und eine kubische körperzentrierte β-Kristallgitterphase von 30 Vol.-% bis 80 Vol.-% aufweist, wobei der Ingot eine Bruch-Zugfestigkeit von 940 MPa oder mehr und einen E-Modul von 150 GPa oder weniger aufweist und in der festen Lösung der Metalle eine glasartige Siliziumkeramik enthält.

11. Verfahren nach Anspruch 10, das ferner den weiteren Schritt umfasst, bei dem der Ingot zu einem medizinischen Implantat geformt wird, das eine Form annimmt, die aus der Gruppe ausgewählt wird, die eine Schraube, einen Stift, einen Stab, eine Stange, eine Feder, eine Spule, ein Seil, eine Klammer, eine Schelle und eine Platte umfasst.

12. Verfahren nach Anspruch 11, das ferner den weiteren Schritt umfasst, bei dem das medizinische Implantat getempert wird.

13. Verfahren nach einem der Ansprüche 10 bis 12, das ferner den weiteren Schritt umfasst, bei dem der Ingot getempert wird.

## Revendications

1. Lingot comprenant un alliage, l'alliage comprenant de 5 % en poids à 35 % en poids de niobium, de 0,5 % en poids à 3,5 % en poids de silicium et de 61,5 % en poids à 94,5 % en poids de titane comme reste, l'alliage n'ayant pas plus de 2 % en poids d'azote, pas plus de 2 % en poids d'oxygène et pas plus de 2 % en poids de carbone, l'alliage ayant une phase α de réseau cristallin hexagonal de 20 % en vol. à 70 % en vol., et une phase de réseau cristallin cubique centré β de 30 % en vol. à 80 % en vol., le lingot ayant une résistance limite à la rupture supérieure ou égale à 940 MPa, et un module de Young inférieur ou égal à 150 GPa, et dans la solution solide des métaux comprenant une céramique de silicium vitreuse.

2. Lingot selon la revendication 1, dans lequel l'alliage comprend de 10 % en poids à 20 % en poids de niobium, de 1,25 % en poids à 2 % en poids de silicium et de 78 % en poids à 88,75 % en poids de titane.

3. Lingot selon l'une quelconque des revendications 1 et 2, qui a une résistance limite à la rupture de 1 000 MPa à 1 400 MPa, et un module de Young de 100 GPa à 150 GPa.

4. Lingot selon la revendication précédente, qui a une résistance limite à la rupture de 1 100 MPa à 1 300 MPa, et un module de Young de 110 GPa à 140 GPa.

5. Lingot selon l'une quelconque des revendications 1 à 4, dans lequel l'alliage a 1 % en poids d'azote ou moins, 1 % en poids d'oxygène ou moins et 1 % en poids de carbone ou moins.

6. Lingot selon la revendication 5, dans lequel l'alliage a 0,5 % en poids d'azote ou moins, 0,5 % en poids d'oxygène ou moins et 0,5 % en poids de carbone ou moins.

7. Lingot selon l'une quelconque des revendications 1 à 6, dans lequel l'alliage comprend une phase α de réseau cristallin hexagonal de 40 % en vol. à 70 % en vol., et une phase de réseau cristallin cubique centré β de 30 % en vol. à 60 % en vol.

8. Implant médical formé à partir d'un lingot selon l'une quelconque des revendications 1 à 7.

9. Implant médical selon la revendication 8, qui est sous la forme d'une vis, d'une broche, d'une tige, d'une barre, d'un ressort, d'un serpentin, d'un câble, d'une agrafe, d'un clip ou d'une plaque.

10. Procédé de formation d'un lingot qui comprend la formation d'un alliage fondu comprenant un mélange uniforme de 5 % en poids à 35 % en poids de niobium, de 0,5 % en poids à 3,5 % en poids de silicium et de 61,5 % en poids à 94,5 % en poids de titane comme reste, l'alliage n'ayant pas plus de 2 % en poids d'azote, pas plus de 2 % en poids d'oxygène et pas plus de 2 % en poids de carbone, le moulage de l'alliage en une forme, et ensuite le refroidissement de la forme en un lingot, l'alliage ayant une phase α réseau cristallin hexagonal de 20 % en vol. à 70 % en vol. et une phase de réseau cristallin cubique centré β de 30 % en vol. à 80 % en vol., le lingot ayant une résistance à la rupture supérieure ou égale à 940 MPa, et un module de Young inférieur ou égal à 150 GPa, et dans la solution solide des métaux comprenant une céramique de silicium vitreuse.

11. Procédé selon la revendication 10, comprenant en outre l'étape ultérieure de formation du lingot en un implant médical sous une forme choisie dans le groupe d'une vis, d'une broche, d'une tige, d'une barre, d'un ressort, d'un serpentin, d'un câble, d'une agrafe, d'un clip et d'une plaque.

12. Procédé selon la revendication 11, comprenant en outre l'étape ultérieure de recuit de l'implant médical.

13. Procédé selon l'une quelconque des revendications 10 à 12, comprenant en outre l'étape ultérieure de recuit du lingot.
